Europäisches Patentamt

European Patent Office

Office européen des brevets

(11) Veröffentlichungsnummer: **0 409 057 A1**

(12) **EUROPÄISCHE PATENTANMELDUNG**

(21) Anmeldenummer: **90113203.5**

(22) Anmeldetag: **11.07.90**

(51) Int. Cl.⁵: **A61M 5/158**, A61M 5/32, A61M 1/00

(30) Priorität: **20.07.89 CH 2705/89**

(43) Veröffentlichungstag der Anmeldung:
**23.01.91 Patentblatt 91/04**

(84) Benannte Vertragsstaaten:
**DE FR GB**

(71) Anmelder: **ROTKREUZSTIFTUNG ZENTRALLABORATORIUM BLUTSPENDEDIENST SRK**
**Wankdorfstrasse 10**
**CH-3000 Bern 22(CH)**

(72) Erfinder: **Fessler, Markus**
**Dorfstrasse 39**
**CH-3423 Ersigen(DE)**

(74) Vertreter: **Keller, René, Dr. et al**
**Patentanwälte Dr. René Keller & Partner**
**Postfach 12 12**
**CH-3000 Bern 7(CH)**

(54) **Vorrichtung für die Zufuhr von Injektionsflüssigkeit oder für die Wegführung von Körperflüssigkeit.**

(57) Eine Injektionsvorrichtung (1) mit einem Nadel (5) haltenden Grundkörper (3) für die Zufuhr von Injektionsflüssigkeit oder für die Wegführung bzw. Aufnahme von Körperflüssigkeit hat ein Nadelschutzelement (7), welches aus seiner Bereitschaftlage, in der die Nadel (5) frei ist, in Richtung vom Nadelschaft (6) zur Nadelspitze (18) ein eine Schutzlage schiebbar ist, wobei dann die Nadelspitze (18) vollständig umhüllt ist. Die Hände des Bedienpersonals befinden sich immer hinter der Nadelspitze (18), wodurch eine Verletzung durch Stechen mit der Nadelspitze (18) unmöglich ist. Auch bei einem äußerst unwahrscheinlichem Abrutschen kann keine Verletzung erfolgen, da keine Bewegung in Richtung von der Nadelspitze (18) zum Nadelschaft (6) vor der Nadelspitze (18) durchgeführt wird.

Fig. 1

EP 0 409 057 A1

# VORRICHTUNG FÜR DIE ZUFUHR VON INJEKTIONSFLÜSSIGKEIT ODER FÜR DIE WEGFÜHRUNG VON KÖRPERFLÜSSIGKEIT

Die Erfindung betrifft eine Vorrichtung für die Zufuhr von Injektionsflüssigkeit oder für die Wegführung bzw. Aufnahme von Körperflüssigkeit mit einen eine Nadel haltenden Grundkörper und mit einem Nadelschutzelement, gemäß dem Oberbegriff des Patentanspruchs 1.

Die Vorrichtungen dienen zum Einführen von Flüssigkeit in den Körper eines Lebewesens, insbesondere eines Menschens und zur Entnahme des sen Körperflüssigkeit, insbesondere zur Blutentnahme. Die Nadeln der bekannten Vorrichtungen haben eine Nadelschutzkappe. Diese dient im wesentlichen zum mechanischen und hygienischen Schutz der Nadel und wird vor dem Gebrauch der Vorrichtung von der Nadel abgezogen. Nach der Durchführung der Injektion bzw. Entnahme von Körperflüssigkeit wird die Nadelschutzkappe wieder über die Nadel gesteckt, um Verletzungen durch die freie Nadel zu vermeiden.

Beim Aufstecken der Nadelschutzkappe muß die Nadel in eine Öffnung im Nadelschutz gleichsam "eingefädelt" werden. Hierbei kann es zu Stichverletzungen an den Händen und/oder Fingern des medizinischen Personals kommen, falls die Öffnung des Nadelschutzes verfehlt wird.

Mit dem Auftreten des HIV (Human Immunodeficiency Virus) hat die Fatalität einer Verletzung durch möglicherweise infizierte Nadeln beim medizinischen Personal zu stark erhöhter Besorgnis geführt; Nadelstiche gelten als die häufigste Verletzungsgefahr im Gesundheitswesen. Besonders gefährlich sind Nadeln an Schläuchen, die auch oft zu komplizierten, schwierig zu handhabenden Bestekken zusammengestellt sind.

Hier will die Erfindung Abhilfe schaffen. Durch die Erfindung, wie sie im Patentanspruch 1 gekennzeichnet ist, wird die Aufgabe gelöst, eine Vorrichtung zu schaffen, bei der die Nadel derart schützbar ist, daß Verletzungsmöglichkeiten des die Injektionsvorrichtung bedienenden Personals ausgeschlossen sind und der Schutz sofort nach dem Herausziehen der Nadel aus dem Körper des Patienten wirksam ist.

In den Patentansprüchen 2 bis 9 sind bevorzugte Ausführungsformen der erfindungsgemäßen Vorrichtung beschrieben.

Im folgenden wird ein Ausführungsbeispiel der erfindungsgemäß Injektionsvorrichtung anhand von Zeichnungen näher erläutert. Es zeigen:

Fig. 1 eine Injektionsvorrichtung mit einer Nadelschutzkappe,

Fig. 2 die Injektionsvorrichtung unmittelbar nach dem Herausziehen der Nadel aus dem Körper des Patienten in zur **Figur 1** leicht gedrehten Lage, und

Fig. 3 eine Draufsicht auf den Grundkörper bei entferntem Nadelschutzelement in Blickrichtung III in **Figur 2**.

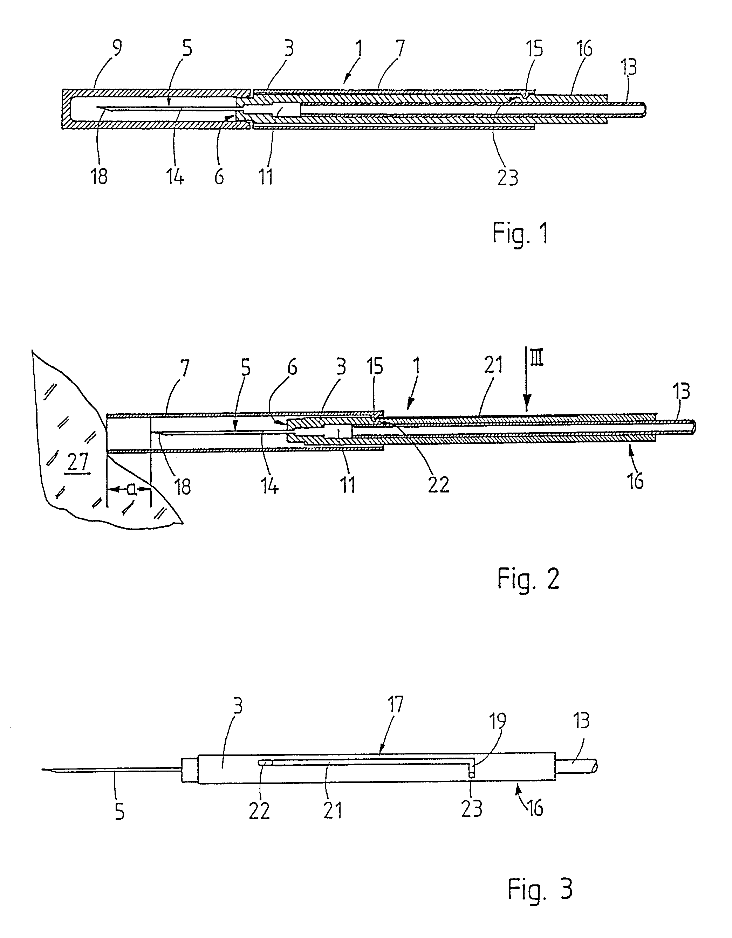

In **Figur 1** ist ein Vorrichtung als Injektionsvorrichtung 1 mit einem zylindrischen Grundkörper 3, in dem eine Nadel 5 mit ihrem Nadelschaft 6 befestigt ist, und der mit einem hülsenförmigen Nadelschutzelement 7 umhüllt ist, dargestellt. Die Oberfläche des Nadelschutzelements 7 ist griffig ausgebildet. Über der Nadel 5 steckt eine zylindrische Nadelschutzkappe 9. Der Grundkörper 3 besitzt eine zentrische Bohrung 11, in der teilweise ein in ihr befestigter Schlauch 13, der ihn an seinem hinteren, der Nadel 5 abgewandten Ende 16 verläßt und zu einer nicht dargestellten Baugruppe, z. B. einem Beute führt. Der schlauchfreie Teil der Bohrung 11 geht im Nadelschaft 6 in die Nadelkanüle 14 über.

Das Nadelschutzelement 7 hat an seiner Innenfläche an seinem der Nadelspitze 18 abgewandten Ende eine Raste 15. In der in **Figur 3** dargestellten Draufsicht auf den Grundkörper 3, wobei das Nadelschutzelement 7 entfernt wurde, ist eine Nut 17 zu sehen, in der die Raste 15 geführt wird. Die Nut 17 beginnt mit einer Umfangsteilnut 19 in der dem Nadelschaft 6 abgewandten Hälfte des Grundkörpers 3. Die Länge der Umfangsteilnut 19 beträgt annähernd ein Drittel des Umfangs des Grundkörpers 3. An diese Umfangsteilnut 19 schließt eine entlang des Mantels des Grundkörpers 3 parallel zu dessen Achse verlaufende Teilnut 21 an, um eine Sicherheitsstrecke a, wie in **Figur 2** dargestellte länger als die Nadel 5 ist. Am der Nadel 5 zugewandten Ende der Teilnut 21 und am dieser abgewandten Ende der Umfangsteilnut 19 befinden sich je eine Vertiefung 22 und 23. Die Vertiefungen 22 und 23 sind derart gestaltet, daß die Raste 15 in sie einrasten kann. In **Figur 1** ist die Raste 15 in der Vertiefung 23 und in **Figur 2** in der Vertiefung 22 eingerastet dargestellt. In **Figur 2** ist die Injektionsvorrichtung 1 gegenüber der in **Figur 1** dargestellten um etwa 120 ° um die Achse des Grundkörpers 3 gedreht.

Der Innendurchmesser des Nadelschutzelements 7 ist so groß gewählt, daß es elastisch leicht verspannt ist, wenn sich die Raste 15 in den vertiefungsfreien Teilen der Teilnuten 19 und 21 befindet und entspannt ist, wenn sich sich in einer der Vertiefung 22 oder 23 befindet. Die elastische Verspannung ist so gewählt, daß sich die Raste 15 aus der Vertiefung 22 bzw. 23 nicht selbständig löst, sondern nur durch eine geringfügig erhöhte Kraftaufwendung gelöst werden kann.

Der Grundkörper 3 steht mit seinem der Nadel 5 abgewandten hinteren Ende 16 über das Nadelschutzelement 7 hinaus. Die Länge des hinteren, herausstehenden Endes 16 wurde so groß gewählt, daß es mit den Fingern gut greifbar ist; seine Oberfläche ist griffig ausgebildet.

Im noch unbenützten Zustand ist die Nadel 5 die Injektionsvorrichtung 1 mit der Nadelschutzkappe 9 abgedeckt und das Nadelschutzelement 7 mit seiner Raste 15 in der Vertiefung 23 spielfrei eingerastet. Soll eine Injektion vorgenommen werden, wird die Injektionsvorrichtung 1 mit dem Nadelschutzelement 7 in die eine Hand genommen, mit der anderen Hand die Nadelschutzkappe 9 von der Nadel 5 abgezogen. Die Injektionsvorrichtung 1 liegt mit dem spielfrei am Grundkörper 3 eingerasteten Nadelschutzelement 7 in Bereitschaftslage sicher in der Hand des medizinischen Personals. Die Nadelspitze 18 wird nun durch die Haut 27 in den Körper des Patienten gestoßen. Je nach geforderter Behandlung wird entweder durch den Schlauch 13 eine Injektionsflüssigkeit eingeführt oder eine Körperflüssigkeit abgesaugt.

Nach beendeter Injektion, die Nadel 5 steckt noch im Körper des Patienten, wird das Nadelschutzelement 7 gegenüber dem Grundkörper 3 nach links verdreht, indem das hintere Ende 16 des Grundkörpers 3 mit der einen und das Nadelschutzelement 7 mit der anderen Hand gehalten wird. Hierdurch wird die Raste 15 aus der Vertiefung 23 herausgedrückt und rutscht entlang der Umfangsteilnut 19 in die Teilnut 21. Nun lassen sich Nadelschutzelement 7 und Grundkörper 3 nicht weiter gegeneinander verdrehen.

Anschließend wird das Nadelschutzelement 7 gegen die Injektionsstelle geschoben - die Raste 15 gleitet in der Teilnut 21 - bis es an der Haut 27 des Patienten um die Injektionsstelle ansteht. Die Bewegung wird fortgesetzt, der Grundkörper 3 weiter aus dem Nadelschutzelement 7 und somit die Nadel 5 aus dem Körper des Patienten herausgezogen, bis die Raste 15 in der Vertiefung 22 in der Schutzlage des Nadelschutzelements 7 einrastet. Die Nadel 5 ist noch vor dem Einrasten aus der Injektionsstelle, d. h. der Haut 27 des Patienten vollständig herausgezogen und gleichzeitig vollständig durch das Nadelschutzelement 7 geschützt worden. Die Nadelspitze 18 ist jetzt nach dem Einrasten um die Sicherheitsstrecke a hinter die freie Seite des Nadelschutzelements 7 zurückgezogen und kann nicht mehr berührt werden. Das Nadelschutzelement 7 befindet sich in seiner Schutzlage.

Bei der oben geschilderten Vorgehensweise befinden sich die Hände der die Injektionsvorrichtung 1 verwendenden Person (medizinisches Personal) immer hinter der Nadelspitze 18, wodurch eine Verletzung durch Stechen mit der Nadelspitze

18 unmöglich ist. Auch bei einem äußerst unwahrscheinlichen Abrutschen kann keine Verletzung erfolgen, da keine Bewegung der Hände in Richtung von der Nadelspitze 18 zum Nadelschaft 6 vor der Nadelspitze 18 durchgeführt wird. Da die Nadel 5 schon während des Herausziehens aus dem Körper des Patienten gesichert wird und sie damit nie mehr frei liegt, werden Verletzungen durch Nadelstiche beim medizinischen Personal verunmöglicht.

Anstelle das Nadelschutzelement 7 und das Ende 24 nur griffig durch eine rauhe und/oder gerillte Oberfläche auszuführen, kann eine nicht dargestellte reliefartige den Fingerformen angepaßte Gestaltung der Oberfläche vorgenommen werden. Hierdurch ist eine besonders sichere Handhabe bei der Injektion gegeben, jedoch müssen dann unterschiedliche Gestaltungen für Rechts- und für Linkshänder gewählt werden. Anstelle eines angepaßten Reliefs kann auch nur ein Rand oder eine Erhöhung an dem der Nadelspitze 18 zugewandten Ende der Nadelschutzelements 7 und am hinteren Ende 24 des Grundkörpers 3 angebracht werden.

Anstelle einer formschlüssigen Verbindung von Nadelschutzelement 7 und Grundkörper 3, wie oben geschildert, kann auch eine kraftschlüssige Verklemmung gewählt werden. Eine einfache kraftschlüssige Verbindung läßt sich erreichen, indem auf dem Grundkörper 3 eine zur Raste 15 (nicht dargestellte) analoge Erhöhung etwa am Ort der Vertiefung 22 oder 23 angebracht wird. Der Querschnitt des Nadelschutzelements 7 wird so gewählt, daß dieses mit Selbsthemmung entlang der Grundkörpers 3 verschiebbar ist. Die Selbsthemmung ist derart, daß eine Verschiebung unter leicht erhöhtem Kraftaufwand zwar möglich ist, aber nicht selbständig durch z. B. Berührung oder unachtsames Anstoßen erfolgen kann. Eine kraftschlüssige Verklemmung gibt allerdings keine so hohe Sicherheit wie eine formschlüssigen Verbindung gegen ungewolltes Verschieben und Verdrehen.

Die oben beschriebene Arretierung des Nadelschutzelements 7 mit einer Raste 15 und jeweils einer Vertiefung 22 und 23 ist wieder lösbar. Soll ein Wiederlösen in der Schutzlage unmöglich sein, kann die Vertiefung 22 in Richtung auf die Umfangsteilnut 19 etwas verlängert werden und der verlängerte Teil mit nicht dargestellten in Richtung Nadelspitze 18 zeigenden Zunge abgedeckt werden. Die Raste 15 würde in diesem Fall klinkenförmig ausgebildet, wobei die Klinke in zur Nadelspitze 18 entgegengesetzter Richtung zeigen würde. Ein Zurückziehen des Nadelschutzelements 7 ist nach dem die Zunge überfahren worden ist, nicht mehr möglich, da sich die Zunge in der Klinke verkeilt.

Anstelle die Raste 15 in der Nut 17 zu führen und in den Vertiefungen 22 und 23 zur Arretierung einrasten zu lassen, kann auch auf die Nut 17

verzichtet werden. In diesem Fall läßt sich das Nadelschutzelement 7 gegen Drehbewegungen nicht gesichert leicht über den Grundkörper 3 in Richtung Nadelspitze 18 führen. Zur Arretierung des Nadelschutzelements 7 hat der Grundkörper 3 jeweils einen nicht dargestellten hinteren und vorderen Ring in Umfangsrichtung am Ort an dem ansonsten die Vertiefungen 22 und 23 angeordnet wären. Das Nadelschutzelement 7 hat dann anstelle der Raste 15 eine radial nach außen in eine nicht dargestellte Vertiefung in ihrer Wand auslenkbare an einer federnden Zunge befestigte nicht dargestellten Noppe. In Bereitschaftlage der Injektionsvorrichtung 1 befindet sich die Noppe auf der der Nadelspitze 18 entgegengesetzten Seite des hinteren Ringes und in Schutzlage auf der der Nadelspitze 18 zugewandten Seite des vorderen Ringes. Ohne die Wirkungsweise zu beeinträchtigen, kann die federnde Noppe auch am Grundkörper 3 und die beiden Ringe auch an der Innenseite des Nadelschutzelements 7 angebracht sein. Auch kann eine nicht dargestellte, federnde Noppe und anstelle jedes Ringes eine nicht dargestellte Umfangsnut verwendet werden.

Aus Sicherheitsgründen kann es vorteilhaft sein, ein Zurückziehen des Nadelschutzelements 7 zu verunmöglichen. In diesem Fall lassen sich entweder mehrere sägezahnförmige Ringe in Umfangsrichtung des Grundkörpers 3 anbringen und die Raste 15 bzw. die Noppe als hierzu passende Klinke ausbilden. Auch kann die Nut 21 mit einer Sägezahnreihe versehen werden, welche ein Zurückziehen verhindert.

Anstelle der Vertiefungen 22 und 23 kann auch die Nut 17 so ausgeführt werden, daß eine bajonettartige Verriegelung des Nadelschutzelements 7 am Grundkörper 3 in Bereitschafts- und Schutzlage gegeben ist.

Der Grundkörper 3 und das Nadelschutzelement 7 müssen nicht kreiszylindrisch ausgeführt werden, sondern können jeden beliebigen Querschnitt, z. B. elliptisch aufweisen. Bevorzugt würde in diesem Fall die Variante mit dem vorderen und hinteren Ring bzw. Nut verwendet. Durch diese spezielle Ausgestaltung läßt sich eine ergonomische Form wählen, welche gut in der Hand der behandelnden Person liegt.

Das Nadelschutzelement 7 kann auch bei entsprechend vorbereitetem Grundkörper bei einer Injektionsvorrichtung ohne Schlauch 13, sondern mit Vorratsbehälter, oder bei einer Spritze verwendet werden.

## Ansprüche

1. Vorrichtung (1) für die Zufuhr von Injektionsflüssigkeit oder für die Wegführung bzw. Aufnahme von Körperflüssigkeit mit einem eine Nadel (5) haltenden Grundkörper (3) und mit einem Nadelschutzelement (7), dadurch gekennzeichnet, daß das Nadelschutzelement (7) aus einer Bereitschaftslage, in welcher die Nadel (5) zur Einführung in den Körper eines Patienten frei ist, in Richtung vom Nadelschaft (6) zur Nadelspitze (18) in eine Schutzlage verschiebbar ist, in welcher es die Nadelspitze (18) umhüllt.

2. Vorrichtung (1) nach Anspruch 1, dadurch gekennzeichnet, daß das Nadelschutzelement (7) als Hülse ausgebildet ist.

3. Vorrichtung (1) nach Anspruch 1 oder 2, dadurch gekennzeichnet, daß der hintere Teil (16) des Grundkörpers (3) und wenigstens ein Teil des Nadelschutzelements (7) als abrutschsichere Griffteile ausgebildet sind.

4. Vorrichtung (1) nach einem der Ansprüche 1 bis 3, dadurch gekennzeichnet, daß das Nadelschutzelement (7) in seiner Schutzlage am Grundkörper (3) form- oder kraftschlüssig arretiert ist.

5. Vorrichtung (1) nach einem der Ansprüche 1 bis 4, dadurch gekennzeichnet, daß das Nadelschutzelement (7) in seiner Bereitschafts- und in seiner Schutzlage am Grundkörper (3) form- oder kraftschlüssig arretierbar und die Arretierung zur Bewegung zwischen den beiden Lagen lösbar ist.

6. Vorrichtung (1) nach Anspruch 4 oder 5, dadurch gekennzeichnet, daß das Nadelschutzelement (7) mindestens in seiner Schutzlage am Grundkörper (3) verriegelbar ist.

7. Vorrichtung (1) nach einem der Ansprüche 4 bis 6, dadurch gekennzeichnet, daß das Nadelschutzelement (7) mittels eines Bajonettverschlusses am Grundkörper (3) verriegelbar ist.

8. Vorrichtung (1) nach einem der Ansprüche 1 bis 7, dadurch gekennzeichnet, daß am Grundkörper (3) und am Nadelschutzelement (7) zusammenwirkende Rastelemente vorhanden sind, die nur eine Schiebebewegung des Nadelschutzelements (7) in Richtung vom Nadelschaft (6) zur Nadelspitze (18) zulassen.

9. Vorrichtung (1) nach einem der Ansprüche 1 bis 8, gekennzeichnet durch eine Nadelschutzkappe (9), die die Nadel (5) der noch unbenütztem Vorrichtung (1) umhüllt und vor der Benützung über die Nadelspitze (18) abziehbar ist.

Fig. 1

Fig. 2

Fig. 3

**Europäisches Patentamt**

## EUROPÄISCHER RECHERCHENBERICHT

EP 90113203.5

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int Cl⁵) |
|---|---|---|---|
| X | US - A - 4 743 233<br>(M.B. SCHNEIDER)<br>  * Fig. 1,4,5; Spalte 2, Zeilen 21,22; Spalte 3, Zeilen 6-35 *<br>-- | 1-8 | A 61 M 5/158<br>A 61 M 5/32<br>A 61 M 1/00 |
| X | US - A - 4 655 751<br>(J.T. HARBAUGH)<br>  * Gesamt; insbesondere Fig. 1-4; Spalte 2, Zeile 27 - Spalte 3, Zeile 52 *<br>-- | 1-6,8, 9 | |
| X | US - A - 4 693 708<br>(A.A. WANDERER et al.)<br>  * Gesamt * | 1,2, 4-8 | |
| A | | 3,9 | |
| X | US - A - 4 826 490<br>(P.O. BYRNE et al.)<br>  * Fig. 1A-2B,5A-5C; Spalte 2, Zeile 63 - Spalte 3, Zeile 13; Spalte 3, Zeilen 27-50 * | 1-4,6, 8 | |
| A | -- | 5 | RECHERCHIERTE SACHGEBIETE (Int Cl⁵)<br><br>A 61 M |
| X | US - A - 4 723 943<br>(J.E. SPENCER)<br>  * Gesamt * | 1-4, 6-8 | |
| A | -- | 5 | |
| X | US - A - 4 826 491<br>(J.J. SCHRAMM)<br>  * Gesamt * | 1,2, 4-6 | |
| A | -- | 3 | |
| X | US - A - 4 747 837<br>(M.W. HAUCK)<br>  * Fig. 1,2; Spalte 2, Zeilen 23-66 * | 1,2,4, 6,9 | |
| A | -- | 3,5 | |

Der vorliegende Recherchenbericht wurde für alle Patentansprüche erstellt.

| Recherchenort | Abschlußdatum der Recherche | Prüfer |
|---|---|---|
| WIEN | 07-11-1990 | LUDWIG |

Europäisches
Patentamt

## EINSCHLÄGIGE DOKUMENTE

| Kategorie | Kennzeichnung des Dokuments mit Angabe, soweit erforderlich. der maßgeblichen Teile | Betrifft Anspruch | KLASSIFIKATION DER ANMELDUNG (Int Cl⁵) |
|---|---|---|---|
| X | <u>US - A - 2 571 653</u><br>(V.G. BASTIEN)<br>* Gesamt * | 1,2,<br>4-6 | |
| A | | 3 | |
| | ---- | | |

RECHERCHIERTE
SACHGEBIETE (Int Cl⁵)

Der vorliegende Recherchenbericht wurde für alle Patentansprüche erstellt

| Recherchenort | Abschlußdatum der Recherche | Prüfer |
|---|---|---|
| WIEN | 07-11-1990 | LUDWIG |